(19) Europäisches Patentamt
European Patent Office
Office européen des brevets

(11) Veröffentlichungsnummer: **0 197 283**
**B1**

(12) # EUROPÄISCHE PATENTSCHRIFT

(45) Veröffentlichungstag der Patentschrift: **21.06.89**

(51) Int. Cl.⁴: **C07C 43/15**, C07C 103/58, C07D 307/48, C07C 41/28, C07C 103/365

(21) Anmeldenummer: **86102621.9**

(22) Anmeldetag: **28.02.86**

(54) **Verfahren zur Herstellung von ungesättigten Verbindungen durch Eliminierunsreaktion.**

(30) Priorität: **02.03.85 DE 3507378**

(43) Veröffentlichungstag der Anmeldung: **15.10.86 Patentblatt 86/42**

(45) Bekanntmachung des Hinweises auf die Patenterteilung: **21.06.89 Patentblatt 89/25**

(84) Benannte Vertragsstaaten: **BE CH DE FR GB IT LI NL**

(56) Entgegenhaltungen:
**EP-A- 0 170 202**
**AT-B- 332 364**
**GB-A- 2 091 259**

**PATENT ABSTRACTS OF JAPAN, Band 8, Nr. 218 (C-245)[1655], 4. Oktober 1984; & JP - A - 59 104 338 (TEIJIN) 16.06.1984**

(73) Patentinhaber: **BASF Aktiengesellschaft, Carl-Bosch-Strasse 38, D-6700 Ludwigshafen(DE)**

(72) Erfinder: **Dockner, Toni, Dr., Grossgasse 6, D-6701 Meckenheim(DE)**
Erfinder: **Hickmann, Eckhard, Dr., Kantstrasse 23, D-6701 Dannstadt-Schauernheim(DE)**
Erfinder: **Krug, Herbert, Mussbacher Strasse 49, D-6700 Ludwigshafen(DE)**

**EP 0 197 283 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von ungesättigten Verbindungen durch Eliminierungsreaktion aus Acetalen oder α-Alkoxiamiden in flüssiger Phase in einem hochsiedenden Mineralöl, wobei das Mineralöl erneuert und das mit Nebenprodukten angereicherte Mineralöl der Verfeuerung zugeführt wird.

Aus AT-B 332 364 ist bekannt, daß man N-Vinylcarbonsäureamide durch Erhitzen von N-α-Alkoxyethylcarbonsäureamiden auf 300 bis 600°C und anschließender rascher Abkühlung des Gasgemisches in einer inerten Flüssigkeit kondensiert und aus dem Kondensat das Produkt isoliert.

In Gegenwart starker Säuren läßt sich z.B. aus Acetalen in flüssiger Phase häufig sehr glatt Alkohol abspalten. Dazu sind hohe Konzentrationen an starker Säure wie Schwefelsäure oder Phosphorsäure erforderlich, um ausreichende Reaktionsgeschwindigkeiten zu erzielen. Die Abspaltungsreaktion verläuft in der Regel bei Temperaturen von 180 bis 200°C in flüssiger Phase. Infolge dieser drastischen Bedingungen entstehen erhebliche Mengen an Nebenprodukten, z.B. durch Veretherung, Isomerisierung und Polymerisation des gebildeten Olefins.

Es bestand daher die Aufgabe, ein Verfahren vorzuschlagen, das die genannten Nachteile nicht aufweist und das es in einfacher Weise gestattet, die Nebenprodukte abzutrennen.

Diese Aufgabe wurde mit einem Verfahren zur Herstellung von Verbindungen der Formel I

$R^1-CH=CH-R^2$ I

in der $R^1$ ein Wasserstoffatom oder einen Alkylrest und $R^2$ einen Alkoxi- oder einen Acylaminorest bedeutet, wobei die Reste $R^1$ und $R^2$ zusammen einen gegebenenfalls substituierten heterocyclischen Ring bilden können, durch Abspaltung des Restes ROH aus Verbindungen der Formel

$$R^1-CH_2-\underset{|}{\overset{R^2}{C}}H-OR^3 \qquad II$$

in der $R^1$ und $R^2$ die oben genannte Bedeutung haben und $R^3$ einen Alkylrest bedeutet, erfindungsgemäß dadurch gelöst, daß man die Abspaltungsreaktion durch Einleiten der Verbindungen der Formel II in flüssigem oder gasförmigem Zustand in ein hochsiedendes Mineralöl bei Temperaturen oberhalb des Siedepunkts der sich bildenden Verbindung I durchführt, die Verbindung I gasförmig abzieht, das hochsiedende Mineralöl bei Anreicherung mit Nebenprodukten erneuert und das mit Nebenprodukten angereicherte hochsiedende Mineralöl abzieht.

Als Verbindungen der Formel II kommen vor allem Acetale und α-Alkoxiamide in Betracht, bei denen der Rest $R^1$ Wasserstoff oder ein aliphatischer Rest mit 1 bis 20, vorzugsweise 1 bis 10 Kohlenstoffatomen ist, der Rest $R^2$ ein gegebenenfalls substituierter Alkoxirest mit 1 bis 20, vorzugsweise 1 bis 10 Kohlenstoffatomen oder ein am Stickstoffatom gegebenenfalls zusätzlich durch Alkyl-, Aralkyl- oder Arylgruppen substituierter Acylaminorest mit 1 bis 20, vorzugsweise 1 bis 10 Kohlenstoffatomen ist oder bei denen $R^1$ und $R^2$ zusammen einen Sauerstoff oder Stickstoff enthaltenden, gegebenenfalls substituierten Heterocyclus bilden und bei denen der Rest $R^3$ ein aliphatischer Rest mit 1 bis 20, vorzugsweise 1 bis 10 Kohlenstoffatomen ist.

Im einzelnen kommen als Ausgangsverbindungen II offenkettige Acetale wie Acetaldehyddimethylacetal, Propionaldehyddimethylacetal, Propionaldehyddiethylacetal, Isobutyraldehyddimethylacetal, cyclische Acetale wie 2,5-Dimethoxi-tetrahydrofuran und insbesondere 3-Formyl-2,5-dimethoxi-tetrahydrofuran sowie α-Alkoxiamide wie α-Methoxi-, α-Ethoxi- oder α-Isopropoxiethylformamid, α-Methoxiethyl-ethylformamid und α-Methoxiethylmethylacetamit in Betracht.

Unter hochsiedenden Mineralölen werden hochsiedende Raffinerieprodukte verstanden mit einem Siedepunkt 150°C, wie Gasöl, Vakuumgasöl, Heizöl S, technisches Weißöl, geschmolzenes Paraffinwachs oder aromatisches Kohlenwasserstofföl. Vorteilhaft wird Vakuumgasöl mit einem Siedepunkt von über 200°C, insbesondere mit einem Siedebereich zwischen 350°C und 500°C verwendet.

Die Abspaltungsreaktion kann je nach Art der Reaktion ohne oder vorzugsweise mit Katalysatoren durchgeführt werden. Bei Verwendung von Katalysatoren können sowohl im Mineralöl unlösliche als auch im Mineralöl lösliche Katalysatoren verwendet werden, die demgemäß im Mineralöl gelöst, emulgiert oder suspendiert sind.

Bevorzugt verwendet man saure Katalysatoren wie aliphatische oder aromatische Sulfonsäuren, z.B. Benzolsulfonsäure oder Toluolsulfonsäure, Dodecylbenzolsulfonsäure, Schwefelsäure und Schwefelsäurehalbester wie Alkylschwefelsäure, Phosphorsäure oder deren partiell veresterte Derivate oder Borsäure und deren saure Derivate. Man kann auch Säureanhydride wie Phosphorpentoxid, Schwefeldioxid und Boroxid verwenden.

Die im Mineralöl löslichen Katalysatoren werden dem Mineralöl im allgemeinen in Mengen von 0,01 bis 25 Gew.-%, vorzugsweise 0,1 bis 10 Gew.-%, insbesondere 0,5 bis 5 Gew.-%, bezogen auf das Mineralöl, zugesetzt.

Als suspendierte Katalysatoren kommen Aluminiumoxid, Aluminiumphosphat, Borphosphat, Aluminiumsilikat, Kieselgel, Titanoxid, Heteropolysäuren des Phosphors und Molybdän- und Wolframsäure in Betracht.

Die Eliminierungsreaktion wird im allgemeinen bei Temperaturen von 50 bis 600°C, vorzugsweise 50 bis 550°C, inbesondere 150 bis 350°C, durchgeführt. Im allgemeinen werden Atmosphärendruck oder erhöhter Druck angewendet. Es ist jedoch auch möglich, bei vermindertem Druck zu arbeiten.

Als Reaktoren sind für die Eliminierungsreaktion z.B. Rührkessel geeignet. Vorteilhaft werden jedoch für das neue Verfahren senkrecht angeordnete zylindrische Reaktoren wie Glockenbodenkolonnen, Blasensäulen oder Füllkörperkolonnen verwendet. Der Ausgangsstoff oder die Ausgangsstoffe werden in der Regel gasförmig oder flüssig am Boden des mit Mineralöl gefüllten Reaktors zugeführt. Es kann vorteilhaft sein, den verdampften Ausgangsstoff mit einem inerten Gas zu verdünnen. Geeignete inerte Gase sind z.B. Wasserdampf, Kohlendioxid und vorzugsweise Stickstoff.

Die Reaktionsprodukte werden gasförmig am Kopf des Reaktors abgezogen. Anschließend werden die gasförmigen Reaktionsprodukte zweckmäßig kondensiert. An die Kondensation kann noch eine Reinigungsstufe, z.B. eine Destillation oder Fraktionierung angeschlossen werden.

Das neue Verfahren kann diskontinuierlich oder kontinuierlich durchgeführt werden, wobei jedoch die kontinuierliche Arbeitsweise bevorzugt wird. Bei der kontinuierlichen Arbeitsweise kann es vorteilhaft sein, das Mineralöl kontinuierlich zuzuführen und abzuziehen, beispielsweise wenn es sich um eine Reaktion handelt, bei der Crackprodukte gebildet werden. Diese Crackprodukte werden dabei mit dem Mineralöl kontinuierlich aus dem Reaktor ausgeschleust. Eine Aufarbeitung und Rückführung des abgezogenen Mineralöls ist in der Regel nicht wirtschaftlich, da das Mineralöl, z.B. als Heizöl oder Vakuumgasöl in der Regel wohlfeil zur Verfügung steht. Man wird daher zweckmäßig das abgezogene Crackprodukt enthaltende Mineralöl einer Verfeuerung und dem Reaktor frisches Mineralöl zuführen.

Das neue Verfahren hat gegenüber demjenigen des Standes der Technik wesentliche Vorteile:

Man kommt mit katalytischen Mengen Säure aus oder kann auch rein thermisch spalten, und die Reaktionstemperatur liegt im allgemeinen 50 bis 100°C tiefer als bei der Eliminierungsreaktion an heterogenen Katalysatoren in der Gasphase. Polymere, Crack- und höhersiedende Nebenprodukte verbleiben im Mineralöl, das nicht regeneriert zu werden braucht und das, gegebenenfalls nach Abtrennen des Katalysators, zweckmäßig dem Kraftwerk zugeführt wird. Bei der Dehydratisierung im "Ölbett" finden keine oder keine nennenswerten Isomerisierungsreaktionen statt und die Ausbeuten liegen höher. Ferner ist das Verfahren in der technischen Ausführung wesentlich wirtschaftlicher als Gasphasenreaktionen an heterogenen Katalysatoren, wie sie in der Literatur zahlreich beschrieben sind und auch in der Praxis ausgeübt werden. Schließlich bewirkt die Verbrennung des Reaktionsmediums und der darin enthaltenen Neben- und Crackprodukte eine verringerte Umweltbelastung.

Die Erfindung wird in den nachfolgenden Beispielen näher erläutert. Teile verhalten sich zu Raumteilen wie Kilogramm zu Liter.

<u>Beispiel 1</u>

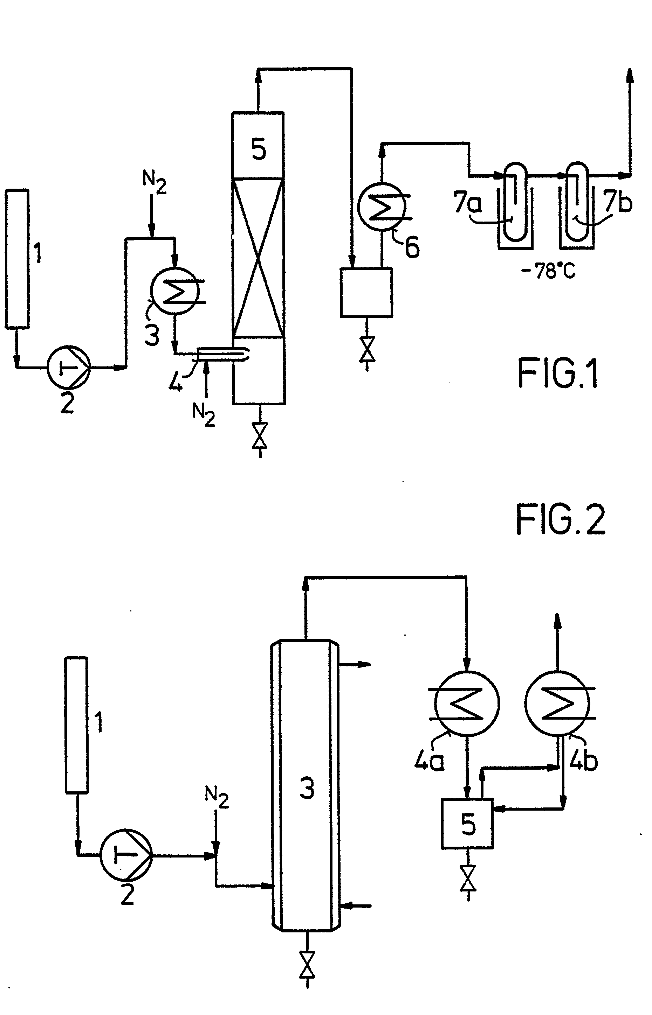

66 Teile Propionaldehyddiethylacetal werden in einer Apparatur gemäß Fig. 1 pro Stunde aus einem Vorratsgefäß 1 über eine Dosierpumpe 2 nach Zuführung von 6000 Raumteilen Stickstoff in einen auf 120°C erhitzten Verdampfer 3 dosiert und von dort gasförmig über eine Zweistoffdüse 4 zusammen mit weiteren 6000 Raumteilen Stickstoff in einen mit 1700 Teilen Vakuumgasöl gefüllten und auf 245°C erhitzten Reaktor 5 geleitet. Der Reaktor ist ein mit Doppelmantel versehenes Glasrohr von 1300 mm Länge und einem Durchmesser von 60 mm, der zu 2/3 mit Pallringen 15 mm gefüllt ist. Das Vakuumgasöl enthält 5 Gew.% Dodecylbenzolsulfonsäure als Katalysator. Die den Reaktor verlassenden Dämpfe werden im Kondensator 6 und in den Kühlfallen 7a und 7b kondensiert. Man erhält stündlich 63 Teile Kondensat mit 2,1 Gew.% nicht umgesetztem Acetal, 46,8 Gew.% Ethyl-1-propenylether und 34 Gew.% Ethanol. Dies entspricht einem Umsatz von 98 %. Die Etherausbeute beträgt 70 % d.Th., bezogen auf umgesetzes Acetal.

<u>Beispiel 2</u>

Man verfährt wie in Beispiel 1, setzt aber 52 Teile Propionaldehyddimethylacetal ein. Man erhält stündlich 48 Teile Kondensat mit 21, 7 Gew.% nicht umgesetztem Acetal, 42 Gew.% Methyl-1-propenylether und 18,7 Gew.% Methanol. Dies entspricht einem Umsatz von 80 % und einer Ausbeute von 70 % d.Th., bezogen auf umgesetztes Acetal.

<u>Beispiel 3</u>

In einer Apparatur gemäß Fig. 2 werden stündlich 72 Teile 2,5-Dimethoxi-3-formyltetrahydrofuran aus der Vorlage 1 mittels Dosierpumpe 2, zusammen mit 200 Raumteilen/h Stickstoff dem Reaktor 3 über ein Kapillarrohr in flüssiger Form zugeführt. Der Reaktor besteht aus einem Doppelmantelrohr von 1,25 m Länge mit einem Durchmesser von 60 mm. Im Reaktor befinden sich keine Einbauten oder Füllkörper. Der Reaktor ist gefüllt mit 1700 Teilen Vakuumgasöl Kp > 350°C, mit 1 Gew.% Dodecylbenzolsulfonsäure als Katalysator und die Reaktionstemperatur beträgt 180°C. Die den Reaktor verlassenden Dämpfe werden in den Kühlern 4a, 4b kondensiert und im Austragsbehälter 5 gesammelt. Um gebildete Nebenprodukte wie z.B. Polymere aus dem Reaktor auszutragen, werden stündlich 100 Teile Vakuumgasöl am Bodenablaßventil ausgeschleust und durch 100 Teile Frischöl mit Katalysator ersetzt. Man erhält stündlich 59,1 Teile Austrag mit 52, 1 Gew.% 3-Formylfuran, 43,2 Gew.% Methanol, 1,3 Gew.% 2,5-Dimethoxi-3-formyltetrahydrofuran und 3 Gew.% 3-Formylfurandimethylacetal. Dies entspricht einem Umsatz von 99 % und einer Ausbeute von 72 % an 3-Formylfuran, bezogen auf den Umsatz

Das gebildete 3-Formylfuran kann durch fraktionierte Destillation in einer Reinheit von 96-98 % bei $Kp_{10}$ = 45°C gewonnen werden.

Vergleichsbeispiel (zu Beispiel 3)

Beim Versuch, aus 2,5-Dimethoxi-3-formyltetrahydrofuran säurekatalysiert unter Verwendung von anorganischen (z.B. Schwefelsäure, Phosphorsäure, saures Kieselgel) oder organischen Säuren (z.B. p-Dodecylbenzolsufonsäure, 2-Ethylhexansäure) unter Methanolabspaltung 3-Formylfuran herzustellen, verharzte der größte Teil des Ausgangsmaterials in exothermer Reaktion. Die maximal erzielbare Ausbeute an 3-Formylfuran betrug ca. 5 % d. Th.

Beispiele 4 bis 6

Zu einem auf ca. 190°C erhitzten Gemisch aus hochsiedendem Paraffinöl ("Weißöl") und 1 % p-Dodecylbenzolsulfonsäure tropft man unter Durchleiten von ca. 20 Volumenteilen Stickstoff innerhalb von ca. 3 bis 5 Stunden ein α-Alkoxiethyl-ethylformamid der allgemeinen Formel

$$H_3C - \underset{\displaystyle CH}{\overset{\displaystyle OR}{|}} - \underset{\displaystyle N}{\overset{\displaystyle CHO}{|}} - CH_2 - CH_3$$

Dabei wird das Ausgangsmaterial vollständig umgesetzt und es destilliert ein Gemisch aus Vinyl-ethylformamid und dem betreffenden Alkohol ROH ab, das nach Kondensation und Redestillation ein Vinylethylformamid in 98,5 bis 99,5 %iger Reinheit liefert.

| Beispiel | α-Alkoxiethylethylformamid R / [Teile] | Weißöl [Raumteile] | p-Dodecylbenzolsulfonsäure [Teile] | Ausbeute an Vinylethylformamid [Teile / % d. Th.] |
|---|---|---|---|---|
| 4 | $CH_3$ / 786 | 400 | 8 | 534 / 90 |
| 5 | $C_2H_5$ / 145 | 400 | 1,5 | 91 / 91 |
| 6 | i-$C_3H_7$ / 79,5 | 400 | 0,9 | 48 / 97 |

**Vergleichsbeispiel (zu Beispiel 6)**
**(analog J. Amer. Chem. Soc. 1982, 104, 6697 und Synthesis 1976, 545)**

Vergleichsbeispiel (zu Beispiel 6) (analog J.Amer.Chem.Soc. 1982, 104, 6697 und Synthesis 1976, 545)

Unter Durchleiten von Stickstoff erhitzte man das Gemisch aus 80 Teilen α-i-Propoxiethyl-ethylformamid und 8 Teilen Ammoniumchlorid ca. 2,5 Stunden auf 140 bis 155°C. Dabei destillierten 24 Teile Niedersieder ab (i-Propanol-haltig). Destillation des Reaktionsrohproduktes lieferte 34 Teile Ethylformamid (identifiziert durch gaschromatographischen Vergleich mit authentischem Ethylformamid, zutreffende Elementaranalyse und NMR-Spektrum), während 29 Teile nichtdestillierbarer Rückstand übrigblieben.

## Patentansprüche

1. Verfahren zur Herstellung von Verbindungen der Formel I
$R^1$–CH=CH–$R^2$ I
in der $R^1$ ein Wasserstoffatom oder einen Alkylrest und $R^2$ einen Alkoxi- oder Acylaminorest bedeutet, wobei die Reste $R^1$ und $R^2$ zusammen einen gegebenenfalls substituierten heterocyclischen Ring bilden können, durch Abspaltung des Restes $R^3$OH aus Verbindungen der Formel II

$$R^1-CH_2-\underset{\displaystyle CH}{\overset{\displaystyle R^2}{|}}-OR^3 \qquad II$$

in der $R^1$ und $R^2$ die oben genannte Bedeutung haben und $R^3$ einen Alkylrest bedeutet, dadurch gekennzeichnet, daß man die Abspaltungsreaktion durch Einleiten der Verbindungen der Formel II in flüssigem

oder gasförmigem Zustand in ein hochsiedendes Mineralöl bei Temperaturen oberhalb des Siedepunkts der sich bildenden Verbindung I durchführt, die Verbindung I gasförmig abzieht, das hochsiedende Mineralöl bei Anreicherung mit Nebenprodukten erneuert und das mit Nebenprodukten angereicherte hochsiedende Mineralöl abzieht.

2. Verfahren gemäß Anspruch 1, dadurch gekennzeichnet, daß man die mit Nebenprodukten angereicherten Mineralöle einer Verfeuerung zuführt.

3. Verfahren gemäß Anspruch 1, dadurch gekennzeichnet, daß man als hochsiedendes Mineralöl Gasöl, Vakuumgasöl, schweres Heizöl, technisches Weißöl oder Vakuumrückstand verwendet.

4. Verfahren gemäß Anspruch 1, dadurch gekennzeichnet, daß man die Abspaltungsreaktion an einem an sich für Dehydratisierungsreaktionen bekannten Katalysator durchführt.

5. Verfahren gemäß Anspruch 1, dadurch gekennzeichnet, daß man die Abspaltungsreaktion kontinuierlich durchführt.

**Claims**

1. A process for the preparation of a compound of the formula I

$R^1-CH=CH-R^2$ I

where $R^1$ is hydrogen or alkyl and $R^2$ is alkoxy or acylamino and $R^1$ and $R^2$ together may form an usubstituted or substituted heterocyclic ring, by elimination of the radical $R^3OH$ from a compound of the formula II

$$R^1-CH_2-CH(R^2)-OR^3 \qquad II$$

where $R^1$ and $R^2$ have the above meanings and $R^3$ is alkyl, wherein the elimination reaction is carried out by passing the compound of the formula II in a liquid or gaseous state into a high-boiling mineral oil at above the boiling point of the compound I being formed, compound I is taken off in gaseous form, and the high-boiling mineral oil enriched with by-products is removed and replaced with fresh mineral oil.

2. A process as claimed in claim 1, wherein the mineral oil enriched with by-products is fed for incineration.

3. A process as claimed in claim 1, wherein the high-boiling mineral oil used is gas oil, vacuum gas oil, heavy fuel oil, industrial white oil or a vacuum residue.

4. A process as claimed in claim 1, wherein the elimination reaction is carried out over a catalyst known per se for dehydration reactions.

5. A process as claimed in claim 1, wherein the elimination reaction is carried out by a continuous procedure.

**Revendications**

1. Procédé de préparation de composés de formule I

$R^1-CH=CH-R^2$ I

dans laquelle $R^1$ représente un atome d'hydrogène ou un radical alkyle et $R^2$ un radical alcoxy ou acylamino, les radicaux $R^1$ et $R^2$ pouvant former ensemble un noyau hétérocyclique éventuellement substitué, par élimination du reste $R^3OH$ dans des composés de formule II

$$R^1-CH_2-CH(R^2)-OR^3 \qquad II$$

dans laquelle $R^1$ et $R^2$ ont les significations données ci-dessus et $R^3$ représente un radical alkyle, caractérisé en ce qu'on conduit la réaction d'élimination par introduction des composés de formule II, à l'état liquide ou gazeux, dans une huile minérale de point d'ébullition élevé à des températures supérieures au point d'ébullition de composé I qui se forme, on extrait le composé I sous forme de gaz, on renouvelle l'huile minérale de point d'ébullition élevé en fonction de son enrichissement en produits secondaires et on soutire l'huile minérale de point d'ébullition élevé enrichie en produits secondaires.

2. Prodédé selon la revendication 1, caractérisé en ce qu'on envoie à la destruction par le feu les huiles minérales enrichies en produits secondaires.

3. Procédé selon la revendication 1, caractérisé en ce qu'on utilise, comme huile minérale de point d'ébullition élevé, du gazole, de l'huile vacuum, du fuel lourd, de l'huile blanche technique ou du résidu sous vide.

4. Procédé selon la revendication 1, caractérisé en ce qu'on conduit la réaction d'élimination en présence d'un catalyseur en soi connu pour des réactions de déshydratation.

5. Procédé selon la revendication 1, caractérisé en ce qu'on mène la réaction d'élimination en continu.

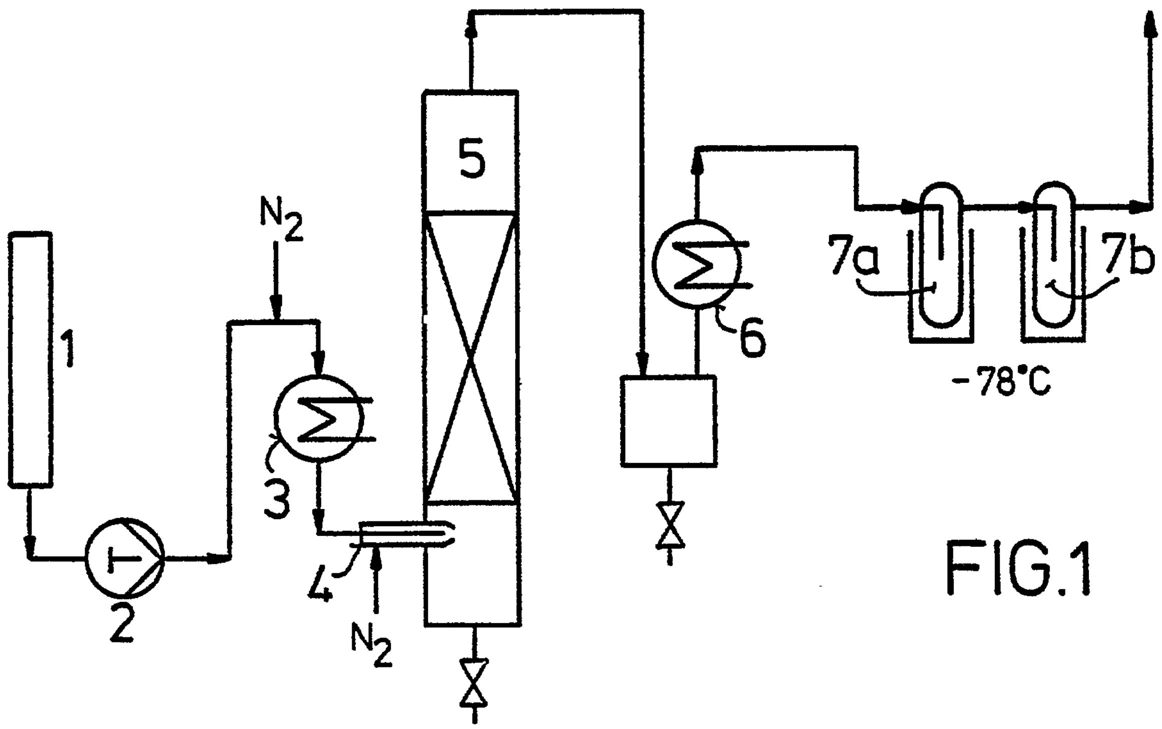


FIG.1

FIG.2